# FASCICULE DE BREVET EUROPEEN

(11) **EP 2 532 385 B1**
(45) Date de publication et mention de la délivrance du brevet: **22.04.2015**
(21) Numéro de dépôt: 11169380.0
(22) Date de dépôt: 09.06.2011
(51) Int. Cl.: A61N 5/10, G21K 1/02

(54) **Dispositif de blindage pour unité d'irradiation**
Abschirmungselement für Strahlungsvorrichtung
Shielding device for irradiation unit

(43) Date de publication de la demande: 12.12.2012
(73) Titulaire: ION BEAM APPLICATIONS S.A., 1348 Louvain-la-Neuve (BE)
(72) Inventeur: Claereboudt, Yves, 1457 Nil-Saint-Vincent (BE); Colmant, Thomas, 4260 Braives (BE); Closset, Michel, 4280 Avin (BE)
(74) Mandataire: Pronovem

(56) Documents cités:
- WO-A1-2010/019504
- WO-A1-2011/060133
- FR-A1- 2 764 199
- US-A- 3 950 651
- US-A- 6 080 992

## Description

### DOMAINE TECHNIQUE

La présente invention se rapporte selon un premier aspect à une méthode d'obtention d'un élément de blindage pour minimiser la pénombre d'un faisceau de hadrons en dehors d'une zone cible, ledit faisceau de hadrons étant issu d'une source et balayé sur ladite zone cible. Selon un second aspect, l'invention se rapporte à un élément de blindage réalisé selon la méthode d'obtention revendiquée. Selon un troisième aspect, la présente invention se rapporte à une unité d'irradiation. Selon un quatrième aspect, la présente invention se rapporte à une méthode de balayage d'une zone cible par un faisceau de hadrons.

### DESCRIPTION DE L'ÉTAT DE LA TECHNIQUE

Les récentes techniques de hadron-thérapie pour le traitement des cancers permettent de délivrer avec précision une dose sur un volume cible, par exemple une tumeur, tout en préservant les tissus environnants. Un appareil de hadron-thérapie comprend un accélérateur de particules produisant un faisceau de particules chargées, un moyen de transport du faisceau et une unité d'irradiation. L'unité d'irradiation délivre une distribution de dose sur le volume cible et comprend généralement des moyens de contrôle de la dose délivrée, comme par exemple une chambre d'ionisation, ainsi que des moyens de contrôle du faisceau, comme par exemple un collimateur ou des aimants de balayage en fonction du mode de délivrance du faisceau employé. Deux grands modes de délivrance de faisceaux sont employés en hadron-thérapie : un premier mode de délivrance comprend les techniques dites de diffusion passive du faisceau et un second mode de traitement plus élaboré comprend les techniques dynamiques de balayage du faisceau.

Les méthodes de diffusion passive font appel à un ensemble d'éléments permettant d'ajuster le trajet des particules jusqu'au point de profondeur maximum de la région à irradier et sur la largeur nécessaire. De manière à obtenir une dose qui coïncide au mieux avec le volume cible, il est nécessaire d'utiliser un compensateur et un collimateur fabriqué spécifiquement pour chaque faisceau du patient. Un défaut majeur de cette technique est que des tissus avoisinants localisés en amont et à l'extérieur du volume cible peuvent être également soumis à des doses élevées de faisceaux. De plus, la fabrication du/des compensateur(s) et du/des collimateur(s) spécifiques à la tumeur du patient et à l'angle d'irradiation est compliquée et coûteuse.

Aussi, une unité d'irradiation d'un appareil de hadron thérapie délivrant un faisceau selon une méthode de diffusion passive comprend un dispositif de fixation de compensateur et collimateur qui lui est propre. Le dispositif de fixation autorise une tranlation le long de l'axe du faisceau afin de placer compensateur et collimateur au plus près de patient. Le collimateur est généralement usiné dans un bloc de laiton aux dimensions adaptées au dispositif de fixation de collimateur, quelle que soit l'ouverture du collimateur. La hauteur du bloc selon la direction du faisceau doit être suffisante pour empêcher le passage du faisceau. Le poids d'un tel collimateur est relativement élevé, ce qui pose des problèmes de manipulation pour le thérapeute. Souvent un tel collimateur est divisé en plusieurs parties suivant sa hauteur pour des facilités de manutention, néanmoins le poids de ces parties de collimateur reste élevé et ces parties requièrent un alignement précis les unes par rapport aux autres. Un collimateur est situé à l'extrémité de l'unité d'irradiation et à une distance proche du patient, et peut avoir un poids total de l'ordre de 30kg, ce qui impose une unité d'irradiation suffisamment robuste ainsi qu'un système de déplacement puissant dans les cas où la gravité doit être vaincue (irradiation verticale).

D'autres collimateurs existent comme par exemple les collimateurs multi-lames tels que décrits dans les documents US20090289192, WO2008106218, dont l'ouverture peut être modifiée en fonction de la forme du volume cible. Ces collimateurs multi-lames sont utilisés pour des techniques de diffusion passive et requièrent des lames qui doivent être longues de façon à couvrir l'ensemble de la surface du faisceau diffusé produit quelle que soit l'ouverture du collimateur demandée et doivent être épaisses de manière à arrêter la totalité du faisceau. De par ces deux besoins, de tels collimateurs multi-lames présentent des désavantages en termes de poids et d'encombrement de l'unité d'irradiation.

Dans un second mode de délivrance de faisceau utilisant des techniques dynamique de balayage du faisceau, comme par exemple la technique du « PBS » (pour Pencil Beam Scanning), décrite dans le document EP1147693, un fin faisceau de particules orienté selon un axe z est balayé selon un plan orthogonal à cet axe z sur le volume cible par le moyen d'aimants de balayage. En faisant varier l'énergie du faisceau de particules, différentes couches dans le volume cible peuvent être irradiées successivement. De cette manière, la dose de radiation peut être délivrée sur l'entièreté du volume cible. Les techniques « PBS » permettent d'irradier un volume cible sans devoir recourir ni à un collimateur, ni à un compensateur d'énergie spécifiques au patient.

La distribution des particules du fin faisceau à la sortie de l'unité d'irradiation suit une distribution gaussienne. La largeur σ du faisceau de hadrons à la sortie de l'unité d'irradiation augmente lorsque l'énergie diminue du fait que la dispersion du faisceau dans les matériaux traversés (détecteurs, air,...) avant la sortie de l'unité d'irradiation varie avec l'énergie du faisceau. Pour des faisceaux d'énergie élevée, la largeur σ du faisceau ne dépasse généralement pas les 3mm. Par contre lorsque l'on diminue l'énergie du faisceau, la largeur σ du faisceau augmente dans certains cas jusque 12mm. Pour des zones où le volume cible avoisine des organes critiques, il est alors nécessaire de pouvoir minimiser la pénombre du faisceau autour du volume cible.

Un dispositif permettant de réduire la pénombre d'un faisceau de protons ou d'ions carbone délivré sur une cible selon une technique de balayage dynamique est décrit dans le document US7826593. Le dispositif est un collimateur multi-lames dans lequel chacune des lames est réalisée en deux parties. Une première partie de lame est réalisée en un matériau de haute densité sélectionné parmi le tungstène, l'osmium et l'iridium, et est assemblée avec une seconde partie réalisée en un matériau plus léger comme de l'acier ou de l'aluminium. Les lames sont disposées selon deux rangées de telle sorte que lesdites premières parties soient situées en vis-à-vis. Bien que l'épaisseur longitudinale selon l'axe du faisceau d'un tel collimateur s'en trouve réduite de part la haute densité des matériaux employés, la mise en oeuvre d'un tel collimateur nécessite l'utilisation de matériaux relativement coûteux, certains de ces matériaux étant difficiles à usiner ou cassants. La mise en oeuvre d'un tel collimateur nécessite aussi la mise au point d'un procédé d'assemblage, par exemple un procédé de brasage de deux plaques en matériaux différents pour former une lame dont le joint entre les deux plaques soit de bonne qualité et uniforme de manière à ce que les lames puissent glisser les unes contre les autres pour former une ouverture.

Un dispositif d'irradiation utilisant un faisceau de protons fixe et un bloc de blindage est divulgué dans le document WO2011/060133 A1.

Le but de la présente invention est de pouvoir réaliser selon une méthode plus simple et moins onéreuse un élément de blindage apte à minimiser la pénombre d'un faisceau de hadrons et présentant des dimensions et un poids réduits.

### RÉSUMÉ DE L'INVENTION

Selon un premier aspect, la présente invention se rapporte à une méthode d'obtention d'un élément de blindage 1 selon la revendication 1.

Plus préférablement, ladite étape de rognage tient compte de la largeur (σ) du faisceau de hadrons 2 de telle sorte que ladite paroi 19 ait une épaisseur latérale en au moins un endroit 8 inférieure à cinq fois la largeur (σ) du faisceau 2.

Selon un mode de réalisation de la méthode de la présente invention, la dite étape de rognage est réalisée de telle sorte à ce que ladite surface externe longitudinale 7b soit semblable à ladite surface interne longitudinale 7a.

Selon un mode de réalisation de la méthode de la présente invention, la méthode comprend une étape de réalisation d'un moyen de support 16 pour fixer ledit élément de blindage 1 dans ladite unité d'irradiation 14.

Selon un premier mode de réalisation dudit moyen de support 16, ledit moyen de support est un rebord situé sur ladite surface externe 7b formé lors de ladite étape de rognage.

Selon un second mode de réalisation dudit moyen de support 16, ledit moyen de support 16 est un socle réalisé en un matériau de basse densité et présentant une ouverture plus grande ou égale à ladite ouverture 11 de forme semblable audit contour 5, 5a et traversant ladite épaisseur longitudinale 6 dudit bloc (13), de manière à laisser passer ledit faisceau 2.

Selon un second aspect, la présente invention se rapporte à un élément de blindage 1 obtenu selon l'un des modes de réalisation de la méthode selon le premier aspect de la présente invention.

Selon un troisième aspect, la présente invention se rapporte à une unité d'irradiation 14 apte à balayer un faisceau de hadrons 2 issu d'une source 3 sur une zone cible 4, ladite unité d'irradiation 14 comprenant un élément de blindage 1 selon le second aspect de la présente invention, positionné entre ladite source 3 et ladite zone cible 4, de manière à minimiser la pénombre du faisceau 2 en dehors de ladite zone cible 4.

### BRÈVE DESCRIPTION DES FIGURES

La figure 1 représente un premier mode de réalisation de la méthode de la présente invention.

La figure 2 représente un second mode de réalisation de la méthode de la présente invention.

La figure 3 représente un troisième mode de réalisation de la méthode de la présente invention.

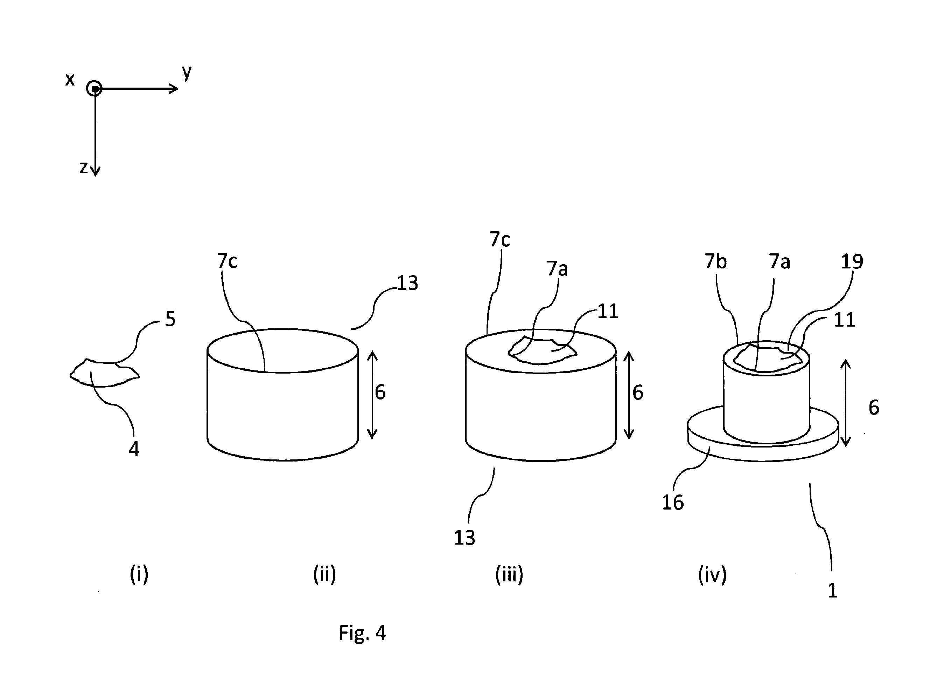

La figure 4 représente un quatrième mode de réalisation de la méthode de la présente invention

La figure 5a représente un élément de blindage selon un second mode de réalisation de la méthode de la présente invention.

La figure 5b représente un élément de blindage selon un premier mode de réalisation de la méthode de la présente invention.

La figure 6 représente une vue tridimensionnelle d'un élément de blindage selon un quatrième mode de réalisation de la méthode de la présente invention.

La figure 7 représente une unité d'irradiation comprenant un élément de blindage selon un second mode de réalisation de la méthode de la présente invention.

La figure 8 représente une unité d'irradiation comprenant un élément de blindage selon un premier mode de réalisation de la méthode de la présente invention.

Les figures sont données à titre indicatif et ne constituent pas de limitation de la présente invention. L'élément de blindage selon la présente invention ayant préférablement une forme particulière en fonction de la zone cible à traiter. Par ailleurs, les proportions des dessins ne sont pas respectées.

### DESCRIPTION DÉTAILLÉE DE L'INVENTION

Selon un premier aspect, la présente invention se rapporte à une méthode d'obtention d'un élément de blindage 1 pour minimiser la pénombre d'un faisceau de hadrons 2 balayé sur une zone cible 4 en dehors de ladite zone cible 4, ledit faisceau de hadrons ayant une largeur gaussienne σ, ledit faisceau étant issu d'une source 3 et orienté substantiellement selon un axe z.

Dans le cas présent, le terme « source » est un point ponctuel à l'origine de la direction prise par le faisceau 2 après avoir été dévié par un premier moyen de balayage 10 selon un axe x et un second moyen de balayage 9 selon un axe y, les deux axes x et y étant orthogonaux entre eux et orthogonaux à un axe z parallèle à la direction du faisceau 2 lorsque celui-ci n'est pas dévié.

Selon un mode d'exécution général de la méthode de la présente invention, la méthode d'obtention d'un élément de blindage 1 comprend les étapes de :
(i) définition d'un contour fermé 5 ou ouvert 5a de ladite zone cible 4 ;
(ii) fourniture d'un bloc 13 ayant une épaisseur longitudinale 6 apte à bloquer le passage du faisceau 2 et ayant une surface latérale 7c perpendiculaire à ladite épaisseur longitudinale 6;
(iii)formation d'une ouverture, par exemple un canal 11 ou une concavité 17 traversant ladite épaisseur longitudinale 6 dudit bloc 13 pour faire passer ledit faisceau, ladite ouverture étant de forme semblable audit contour et formant une surface interne longitudinale 7a;
La méthode comprend une étape supplémentaire (iv) de rognage dudit bloc 13 de manière à former une surface externe longitudinale 7b autour de ladite surface interne longitudinale 7a, lesdites surface interne longitudinale 7a et externe longitudinale 7b délimitant une paroi 19.
Le terme « épaisseur longitudinale » se réfère à une épaisseur selon ledit axe z. Le terme « surface longitudinale » se réfère à une surface parallèle à l'axe z. L'ouverture 11, 17 est définie en fonction de la zone cible à traiter et est de profil semblable à un contour 5 de la zone cible 4 ou à une partie 5a d'un contour 5 de la zone cible 4. Le terme « contour fermé» utilisé dans la présente demande, désigne une ligne fermée sur elle-même et qui délimite une zone cible, le terme «contour ouvert » désigne une ligne ouverte qui délimite une partie d'une zone cible.

Préférablement, le bloc 13 fourni pour la formation de l'élément de blindage 1 est réalisé en un matériau présentant un pouvoir d'arrêt du faisceau suffisamment élevé et s'activant peu lorsque celui-ci est soumis à l'irradiation. Un matériau généralement utilisé est le laiton car il est un bon compromis satisfaisant à ces deux conditions, est peu couteux et est facile à usiner.

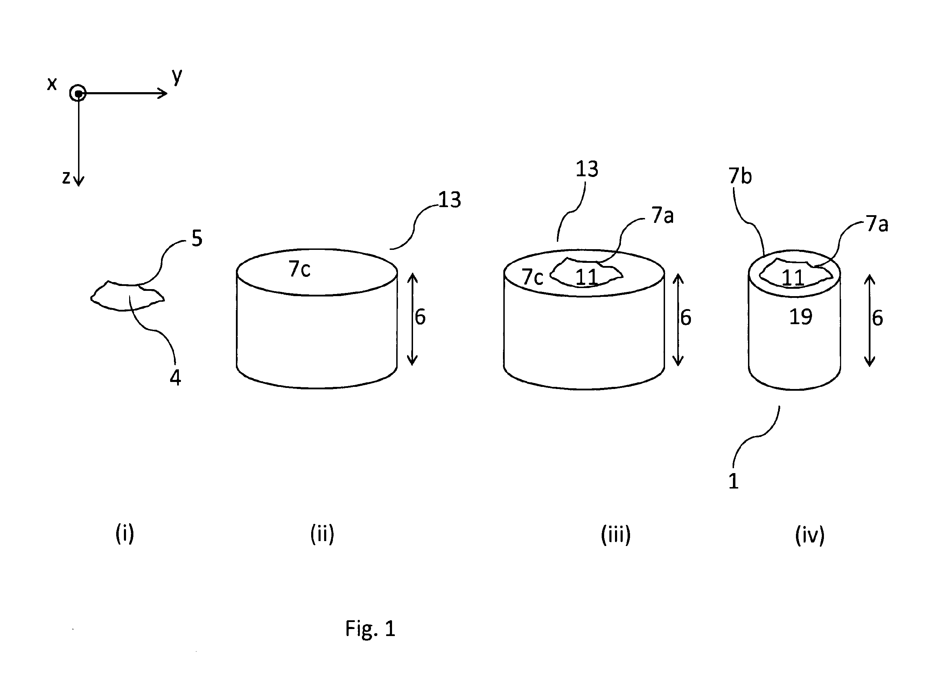

La figure 1 représente un premier mode d'exécution de la méthode selon la présente invention dans lequel le contour de la zone cible défini dans l'étape (i) est un contour fermé 5.

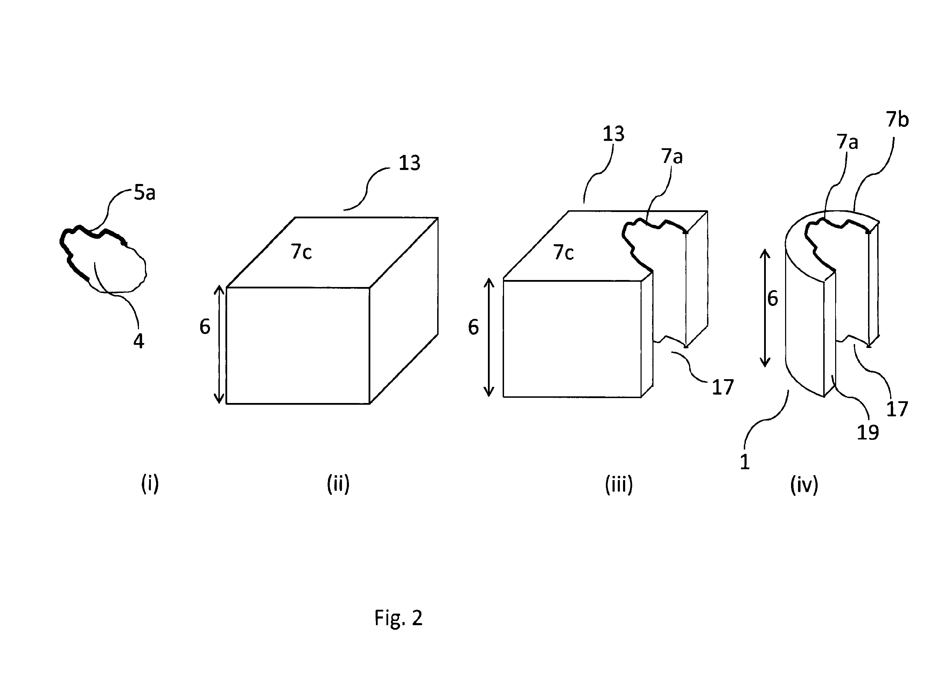

La figure 2 représente un second mode d'exécution de la méthode selon la présente invention dans lequel le contour de la zone cible défini dans l'étape (i) est un contour ouvert 5a.

La méthode d'obtention de l'élément de blindage 1 est caractérisée en ce que ladite étape de rognage (iv) tient compte de la largeur σ du faisceau de hadrons 2 de telle sorte que ladite paroi 19 ait une épaisseur latérale partout supérieure à au moins une fois la largeur σ dudit faisceau. Le terme « épaisseur latérale » se réfère à une épaisseur entre la surface interne longitudinale 7a et la surface externe longitudinale 7b mesurée dans une direction orthogonale audit axe z et perpendiculairement par rapport à la surface interne longitudinale 7a.

Pour un appareil de hadron thérapie utilisant une technique de balayage dynamique d'un faisceau sur une zone cible et comprenant un modulateur d'énergie du faisceau, la largeur σ du faisceau varie en fonction de l'énergie du faisceau entre une valeur minimum σₘᵢₙ ( par exemple 3mm) et une valeur maximum σₘₐₓ (par exemple 12mm). En pratique, l'homme du métier pourra choisir de réaliser ladite étape de rognage (iv) de telle sorte que l'épaisseur latérale de la paroi latérale soit supérieure à trois fois la largeur σ dudit faisceau.

Dans un autre mode d'exécution de la méthode de la présente invention, ladite étape de rognage tient compte de la largeur σ dudit faisceau de telle sorte que ladite paroi 19 ait une épaisseur latérale partout supérieure à au moins une fois la largeur σ dudit faisceau et de telle sorte que ladite paroi 19 ait une épaisseur latérale en au moins un endroit 8 inférieure à cinq fois la largeur σ dudit faisceau.

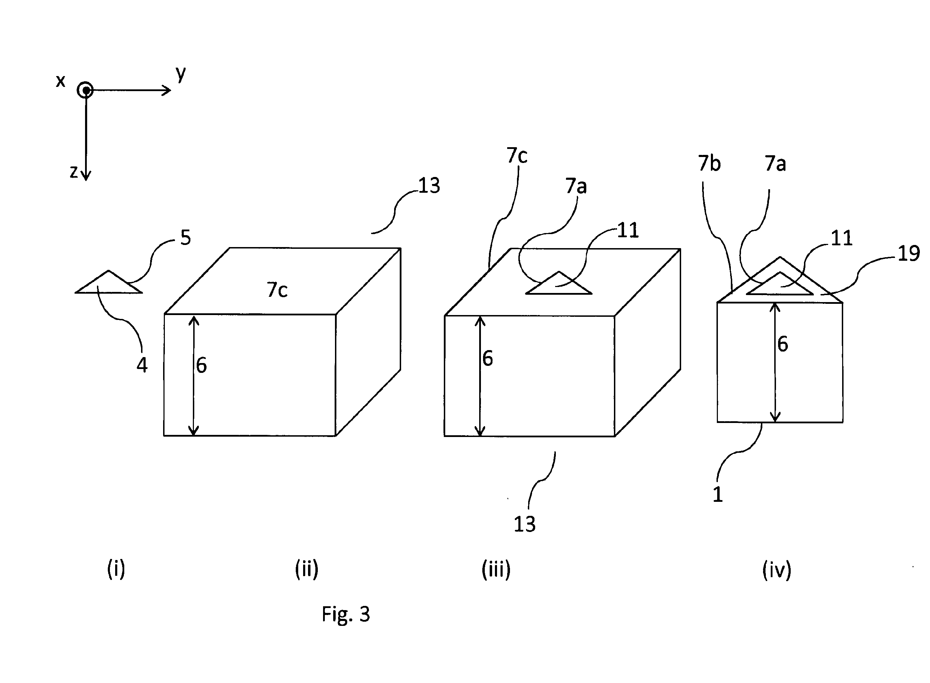

Dans un autre mode d'exécution de la méthode de la présente invention tel que représenté sur la figure 3, ladite surface externe longitudinale 7b formée lors de ladite étape de rognage est semblable à ladite surface interne longitudinale 7a. Préférablement ladite paroi 19 délimitée par lesdites surface interne longitudinale 7a et externe longitudinale 7b a une épaisseur latérale partout supérieure à au moins une fois la largeur σ dudit faisceau. Plus préférablement, ladite paroi 19 a une épaisseur latérale en au moins un endroit 8 inférieure à cinq fois la largeur σ dudit faisceau.

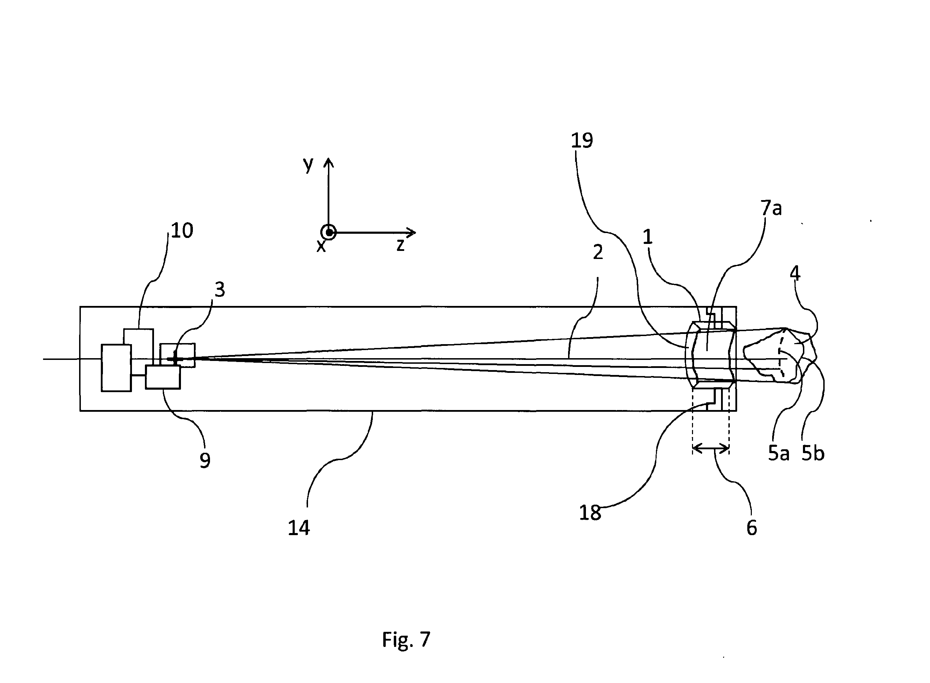

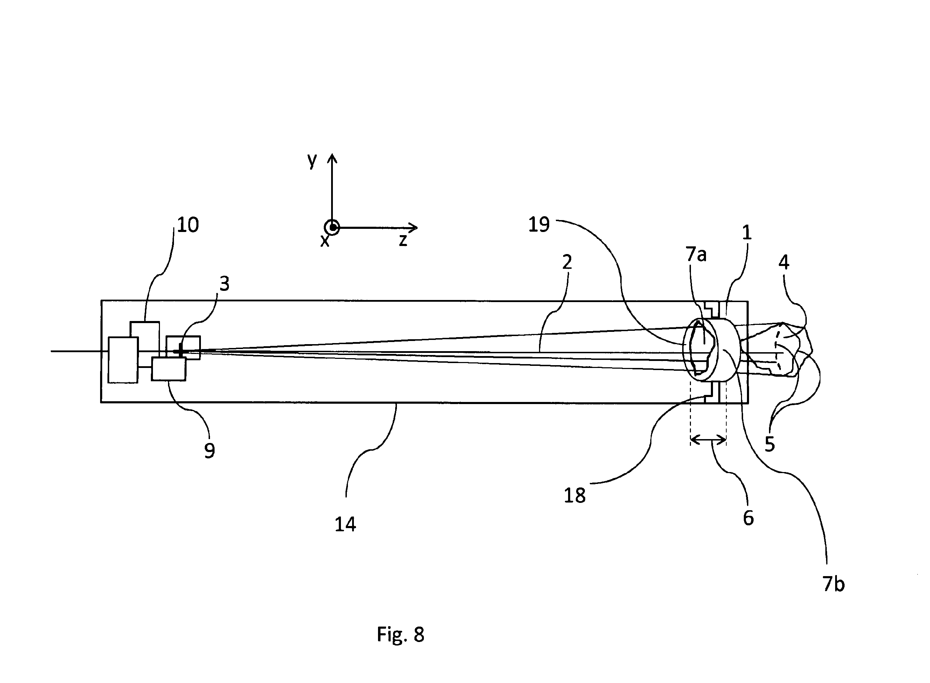

Dans un autre mode d'exécution de la méthode selon l'un quelconque des modes d'exécution précédents, ladite méthode comprend une étape de formation d'un moyen de support 16 pour permettre la fixation de l'élément de blindage 1 dans une unité d'irradiation 14 telle que représentée par exemple sur les figures 7 et 8.

Selon un premier mode de réalisation de l'étape de formation dudit moyen de support, un rebord situé sur la surface externe longitudinale 7b est formé lors de ladite étape de rognage, ledit rebord servant de moyen de support 16. Préférablement, ledit rebord est de dimension adaptée à un dispositif de fixation 18 d'un élément de blindage compris dans une unité d'irradiation 14 telle que représentée par exemple sur les figures 7 et 8, quelque soient la forme de l'ouverture 11, 17 et l'épaisseur latérale de la paroi 19.

Selon un second mode de réalisation de l'étape de formation dudit moyen de support 16, un socle est fixé sur l'élément de blindage 1, ledit socle ayant une ouverture plus grande ou égale à ladite ouverture 11, 17 formée dans ledit bloc 13, de manière à laisser passer le faisceau 2. Préférablement, le socle est de dimensions adaptées à un dispositif de fixation 18 d'un élément de blindage compris dans une unité d'irradiation 14 telle que représentée par exemple sur les figures 7 et 8, quelque soient la forme de l'ouverture 11, 17 et l'épaisseur latérale de la paroi 19. Préférablement, ledit socle est réalisé en un matériau de basse densité, comme par exemple du plexiglas, suffisamment résistant aux radiations et suffisamment robuste pour soutenir le poids de l'élément de blindage.

D'autres procédés de formation d'un moyen de support 16 pour permettre la fixation de l'élément de blindage 1 dans une unité d'irradiation 14 peuvent être envisagés par l'homme de métier.

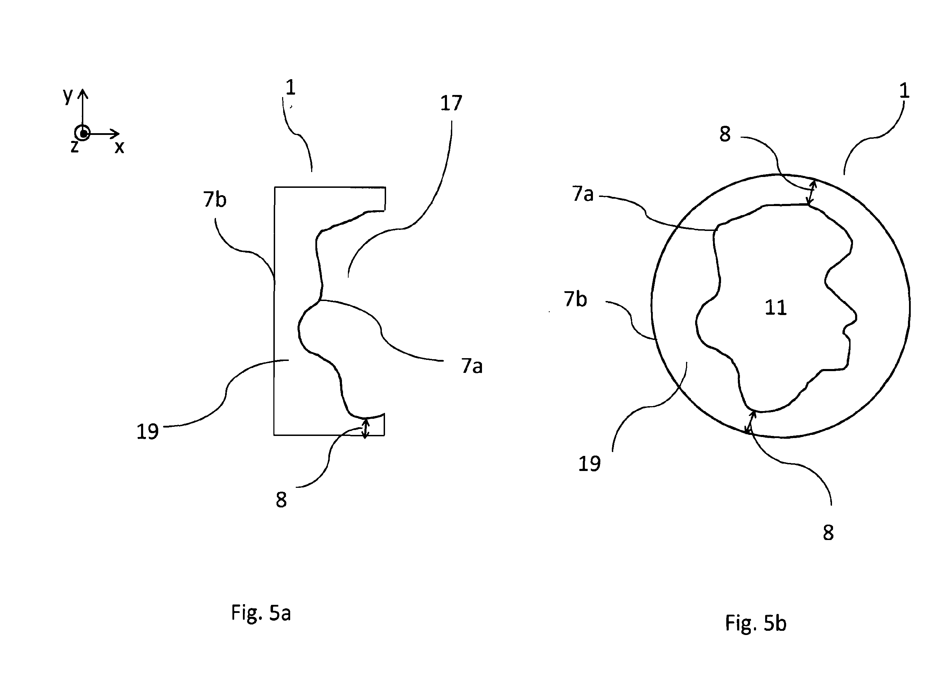

Selon un second aspect, la présente invention se réfère à un élément de blindage 1 obtenu par la méthode de la présente invention. La figure 5a représente une vue suivant l'axe z d'un premier mode de réalisation d'un élément de blindage 1 obtenu selon la méthode de la présente invention. L'élément de blindage comprend une ouverture 17 formant une surface interne longitudinale 7a et autour de laquelle une surface externe longitudinale 7b a été formée par rognage, ladite surface interne longitudinale 7a délimitant avec ladite surface externe longitudinale 7b une paroi latérale 19. Ladite ouverture 17 vue dans le plan xy est de forme semblable à un contour ouvert 5a d'une zone cible 4. L'épaisseur latérale de la paroi 19 est partout supérieure ou égale à au moins une fois la largeur σ du faisceau. Un tel élément de blindage 1 est utilisable pour l'irradiation par une technique de balayage dynamique d'une zone cible 4 dont seulement un côté avoisine une zone critique pour laquelle on souhaite minimiser la pénombre du faisceau. Préférablement, l'épaisseur latérale de la paroi 19 est inférieure en au moins un endroit à un nombre de N fois la largeur σ dudit faisceau, N étant choisi par l'utilisateur et étant avantageusement compris entre 1 et 5 de sorte à ce que le poids de l'élément de blindage soit minimisé tout en ayant une paroi latérale 19 apte à empêcher le passage du faisceau.

La figure 5b représente une vue dans le plan xy d'un second mode de réalisation d'un élément de blindage 1 obtenu selon la méthode de la présente invention. L'élément de blindage 1 comprend une ouverture 11 formant une surface interne longitudinale 7a et autour de laquelle une surface externe longitudinale 7b a été formée par rognage, la surface interne longitudinale 7a délimitant avec la surface externe longitudinale 7b une paroi 19. Ladite ouverture 11, vue dans le plan xy, est de forme semblable à un contour fermé 5 d'une zone cible 4. L'épaisseur latérale de la paroi 19 est partout supérieure à au moins une fois la largeur σ du faisceau. Préférablement, l'épaisseur latérale de la paroi 19 est inférieure en au moins un endroit à un nombre de N fois la largeur σ dudit faisceau, N étant choisi par l'utilisateur et étant avantageusement compris entre 1 et 5 de sorte à ce que le poids de l'élément de blindage soit minimisé tout en ayant une paroi 19 de dimensions aptes à empêcher le passage du faisceau.

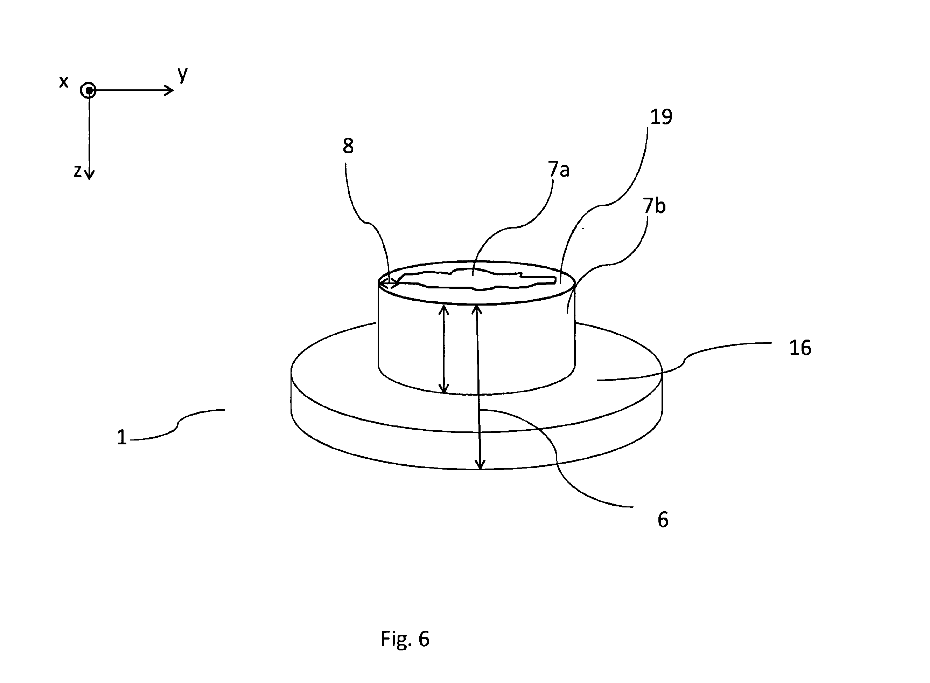

La figure 6, représente une vue tridimensionnelle d'un troisième mode de réalisation d'un élément de blindage 1 obtenu selon la méthode de la présente invention, et comprenant un moyen de support 16, par exemple un rebord ou un socle. L'élément de blindage 1 est réalisée en un matériau de pouvoir d'arrêt élevé et s'activant peu lorsque celui-ci est soumis à l'irradiation, comme par exemple du laiton. Lorsque le moyen de support 16 est un socle, celui-ci peut être également réalisé dans le même matériau que le bloc 13 ayant servi à la réalisation de l'élément de blindage 1, ou dans un autre matériau plus léger, tant que l'épaisseur longitudinale totale 6 de l'élément de blindage soit apte à empêcher le passage du faisceau 2.

Chacun des éléments de blindage tels que décrits ont pour avantage de présenter un volume et un poids réduits tout en permettant de limiter la pénombre d'un faisceau de largeur σ en dehors du contour ouvert ou fermé de la zone cible 4.

Selon un troisième aspect, la présente invention se rapporte à une unité d'irradiation 14 comprenant un élément de blindage 1 selon l'un quelconque des modes de réalisation décrits précédemment.

Préférablement, l'unité d'irradiation 14 fait partie d'un appareil de hadron thérapie apte à délivrer un faisceau 2 sur une zone cible 4 selon une technique de balayage dynamique. L'unité d'irradiation comprend préférablement un premier moyen de balayage 10 du faisceau selon un axe x et un second moyen de balayage 9 du faisceau selon un axe y, les deux axes x et y étant orthogonaux entre eux et orthogonaux à un axe z parallèle à la direction du faisceau 2 lorsque celui-ci n'est pas dévié.

L'unité d'irradiation 14 comprend un moyen d'attache 18 apte à maintenir l'élément de blindage 1 à une distance proche de la zone cible 4 et à une distance éloignée de la source 3. Par exemple, l'élément de blindage 1 se trouve à une distance d'environs 2 ou 3 mètres de la source 3 et à une distance inférieure à 50 cm de la zone cible 4.

La figure 7 représente un exemple non limitatif de la présente invention d'une unité d'irradiation 14 comprenant un élément de blindage 1 selon le premier mode de réalisation dans lequel l'élément de blindage 1 comprend une ouverture de forme semblable à un contour ouvert 5a d'une zone cible 4.

La figure 8 représente un exemple non limitatif de la présente invention d'une unité d'irradiation 14 comprenant un élément de blindage 1 selon le second mode de réalisation dans lequel l'élément de blindage 1 comprend une ouverture 11 de forme semblable à un contour fermé 5 d'une zone cible 4.

L'unité d'irradiation 14 selon la présente invention peut comprendre tout autre mode de réalisation d'un élément de blindage 1 formé selon la méthode de la présente invention.

Préférablement, l'unité d'irradiation 14 comprend à l'extrémité faisant face à ladite zone cible 4 une portion rétractable comprenant l'élément de blindage 1. Une telle portion rétractable permet de rapprocher l'élément de blindage près de la zone cible 4 de manière à délivrer un faisceau avec une meilleure précision, et d'éloigner l'élément de blindage 1 de la zone cible 4 lorsque l'on désire changer l'angle d'inclinaison de l'unité d'irradiation. Dans les unités d'irradiation conventionnelles, la portion rétractable est généralement comprise dans une enceinte à l'air libre comprenant un dispositif de déplacement de la portion rétractable suffisamment robuste, comme par exemple un rail. La diminution du poids de l'élément de blindage 1, par rapport aux collimateurs et collimateurs multi lames de l'art antérieur, permet d'utiliser une portion rétractable télescopique de taille réduite permettant de maintenir le vide dans l'unité d'irradiation 14 sur une plus longue distance et obtenir un faisceau moins diffus.

## Revendications

1. Méthode d'obtention d'un élément de blindage (1) pour minimiser la pénombre d'un faisceau de hadrons (2) en dehors d'une zone cible (4), ledit faisceau de hadrons (2) étant guidé dans une direction longitudinale pour effectuer un balayage sur une zone cible par une unité d'irradiation (14), ledit faisceau (2) ayant une largeur (σ), la méthode comprenant des étapes de :
(i) définition d'un contour fermé (5) ou ouvert (5a) de ladite zone cible (4) ;
(ii) fourniture d'un bloc (13) ayant une épaisseur longitudinale (6) apte à bloquer le passage dudit faisceau (2) et ayant une surface latérale (7c) perpendiculaire à ladite épaisseur longitudinale (6) ;
(iii) formation d'une ouverture (11, 17) de forme correspondant audit contour et traversant ladite épaisseur longitudinale (6) dudit bloc (13) pour faire passer ledit faisceau (2), ladite ouverture (11, 17) formant une surface interne longitudinale (7a);
(iv) rognage dudit bloc (13) de manière à former une surface externe longitudinale (7b) autour de ladite surface interne longitudinale (7a), lesdites surfaces interne longitudinale (7a) et externe longitudinale (7b) délimitant une paroi latérale (19) qui a une épaisseur latérale partout supérieure à au moins une fois la largeur (σ) du faisceau;
(v) fixation dudit élément de blindage (1) dans ladite unité d'irradiation (14) configurée pour effectuer un balayage dudit faisceau de hadrons (2) sur la zone cible.

2. Méthode d'obtention d'un élément de blindage (1) selon la revendication 1 **caractérisée en ce que** ladite étape de rognage tient compte de la largeur (σ) du faisceau de hadrons (2) de telle sorte que ladite paroi (19) ait une épaisseur latérale en au moins un endroit (8) inférieure à cinq fois la largeur (σ) du faisceau (2).

3. Méthode d'obtention d'un élément de blindage selon l'une quelconque des revendications précédentes **caractérisée en ce que** ladite surface externe longitudinale (7b) correspond à ladite surface interne longitudinale (7a).

4. Méthode d'obtention d'un élément de blindage (1) selon l'une quelconque des revendications précédentes **caractérisée en ce qu'**elle comprend une étape de réalisation d'un moyen de support (16) pour fixer ledit élément de blindage (1) dans ladite unité d'irradiation (14).

5. Méthode d'obtention d'un élément de blindage (1) selon la revendication 4 **caractérisée en ce que** ledit moyen de support (16) est un rebord situé sur ladite surface externe longitudinale (7b) formé lors de ladite étape de rognage.

6. Méthode d'obtention d'un élément de blindage (1) selon la revendication 4 **caractérisée en ce que** ledit moyen de support (16) est un socle présentant une ouverture plus grande ou égale à ladite ouverture (11) de forme correspondant audit contour (5, 5a) et traversant ladite épaisseur longitudinale (6) dudit bloc (13), de manière à laisser passer ledit faisceau (2).

7. Méthode d'obtention d'un élément de blindage (1) selon la revendication 6 **caractérisée en ce que** le socle est réalisé en plexiglas.

8. Unité d'irradiation (14) apte à balayer un faisceau de hadrons (2) issu d'une source (3) sur une zone cible (4), ladite unité d'irradiation (14) comprenant un élément de blindage (1) positionné entre ladite source (3) et ladite zone cible (4), de manière à minimiser la pénombre du faisceau (2) en dehors de ladite zone cible (4), ledit élément de blindage étant obtenu selon une méthode comprenant des étapes de :
(i) définition d'un contour fermé (5) ou ouvert (5a) de ladite zone cible (4) ;
(ii) fourniture d'un bloc (13) ayant une épaisseur longitudinale (6) apte à bloquer le passage dudit faisceau (2) et ayant une surface latérale (7c) perpendiculaire à ladite épaisseur longitudinale (6) ;
(iii) formation d'une ouverture (11, 17) de forme correspondant audit contour et traversant ladite épaisseur longitudinale (6) dudit bloc (13) pour faire passer ledit faisceau (2), ladite ouverture (11, 17) formant une surface interne longitudinale (7a);
(iv) rognage dudit bloc (13) de manière à former une surface externe longitudinale (7b) autour de ladite surface interne longitudinale (7a), lesdites surfaces interne longitudinale (7a) et externe longitudinale (7b) délimitant une paroi latérale (19) qui a une épaisseur latérale partout supérieure à au moins une fois la largeur (σ) du faisceau.

9. Unité d'irradiation selon la revendication 8 **caractérisée en ce que** ladite étape de rognage tient compte de la largeur (σ) du faisceau de hadrons (2) de telle sorte que ladite paroi (19) ait une épaisseur latérale en au moins un endroit (8) inférieure à cinq fois la largeur (σ) du faisceau (2).

10. Unité d'irradiation selon l'une des revendications 8 ou 9 **caractérisée en ce que** ladite surface externe longitudinale (7b) correspond à ladite surface interne longitudinale (7a).

11. Unité d'irradiation selon l'une quelconque des revendications 8 à 10 **caractérisée en ce que** ladite méthode comprend une étape de réalisation d'un moyen de support (16) pour fixer ledit élément de blindage (1) dans ladite unité d'irradiation (14).

12. Unité d'irradiation selon la revendication 11 **caractérisée en ce que** ledit moyen de support (16) est un rebord situé sur ladite surface externe longitudinale (7b) formé lors de ladite étape de rognage.

13. Unité d'irradiation selon la revendication 11 **caractérisée en ce que** ledit moyen de support (16) est un socle présentant une ouverture plus grande ou égale à ladite ouverture (11) de forme correspondant audit contour (5, 5a) et traversant ladite épaisseur longitudinale (6) dudit bloc (13), de manière à laisser passer ledit faisceau (2).

14. Unité d'irradiation selon la revendication 13 **caractérisée en ce que** le socle est réalisé en plexiglas.

## Patentansprüche

1. Herstellungsmethode eines Abschirmungselements (1) zur Minimierung des Halbschattens eines Hadronenstrahls (2) außerhalb eines Zielbereichs (4), wobei der Hadronenstrahl (2) in eine Längsrichtung gelenkt ist, um durch eine Strahlungseinheit (14) eine Abtastung in einem Zielbereich durchzuführen (14), wobei der Strahl (2) eine Breite (σ) hat, wobei die Methode Schritte umfasst der:
(i) Definition einer geschlossenen (5) oder geöffneten (5a) Kontur des Zielbereichs (4),
(ii) Bereitstellung eines Blocks (13) mit einer Längsdicke (6), die imstande ist, den Durchgang des Strahls (2) zu blockieren und mit einer Seitenfläche (7c) senkrecht zur Längsdicke (6),
(iii) Bildung einer Öffnung (11, 17) einer Form, die der Kontur entspricht und die Längsdicke (6) des Blocks (13) durchquert, um den Strahl (2) hindurchzuleiten, wobei die Öffnung (11, 17) eine innere Längsfläche (7a) bildet,
(iv) Beschneidung des Blocks (13) derart, dass eine äußere Längsfläche (7b) um die innere Längsfläche (7a) gebildet wird, wobei die innere (7a) und äußere (7b) Längsfläche eine Seitenwand (19) begrenzen, die eine seitliche Dicke hat, die überall größer als mindestens einmal die Breite (σ) des Strahls ist,
(v) Befestigung des Abschirmungselements (1) in der Strahlungseinheit (14), die konfiguriert ist, um den Hadronenstrahls (2) über den Zielbereich zu führen.

2. Herstellungsmethode eines Abschirmungselements (1) nach Anspruch 1, **dadurch gekennzeichnet, dass** der Schritt des Beschneidens die Breite (σ) des Hadronenstrahls (2) derart berücksichtigt, dass die Wand (19) an mindestens einer Stelle (8) eine seitliche Dicke hat, die fünfmal kleiner als die Breite (σ) des Strahls (2) ist.

3. Herstellungsmethode eines Abschirmungselements nach einem der vorangehenden Ansprüche, **dadurch gekennzeichnet, dass** die äußere Längsfläche (7b) der inneren Längsfläche (7a) entspricht.

4. Herstellungsmethode eines Abschirmungselements (1) nach einem der vorangehenden Ansprüche, **dadurch gekennzeichnet, dass** sie einen Schritt der Realisierung eines Stützmittels (16) umfasst, um das Abschirmungselement (1) in der Strahlungseinheit (14) zu befestigen.

5. Herstellungsmethode eines Abschirmungselements (1) nach Anspruch 4, **dadurch gekennzeichnet, dass** das Stützmittel (16) ein während des Schneidschritts gebildeter Rand ist, der sich auf der äußeren Längsfläche (7b) befindet.

6. Herstellungsmethode eines Abschirmungselements (1) nach Anspruch 4, **dadurch gekennzeichnet, dass** das Stützmittel (16) ein Sockel ist, der eine Öffnung aufweist, die größer oder gleich der Öffnung (11) ist mit einer Form, die der Kontur (5, 5a) entspricht und die Längsdicke (6) des Blocks (13) derart durchquert, um den Strahl (2) passieren zu lassen.

7. Herstellungsmethode eines Abschirmungselements (1) nach Anspruch 6, **dadurch gekennzeichnet, dass** der Sockel aus Plexiglas hergestellt ist.

8. Strahlungseinheit (14), die imstande ist, einen Hadronenstrahl (2) aus einer Quelle (3) über einen Zielbereich (4) zu führen, wobei die Strahlungseinheit (14) ein Abschirmungselement (1) umfasst, das zwischen der Quelle (3) und dem Zielbereich (4) derart positioniert ist, um den Halbschatten des Strahls (2) außerhalb des Zielbereichs (4) zu minimieren, wobei das Abschirmungselement nach einer Methode hergestellt ist, das die Schritte umfasst der:
(i) Definition einer geschlossenen (5) oder geöffneten (5a) Kontur des Zielbereichs (4),
(ii) Bereitstellung eines Blocks (13) mit einer Längsdicke (6), die imstande ist, den Durchgang des Strahls (2) zu blockieren und mit einer Seitenfläche (7c) senkrecht zur Längsdicke (6),
(iii) Bildung einer Öffnung (11, 17) einer Form, die der Kontur entspricht und die Längsdicke (6) des Blocks (13) durchquert, um den Strahl (2) hindurchzuleiten, wobei die Öffnung (11, 17) eine innere Längsfläche (7a) bildet,
(iv) Beschneidung des Blocks (13) derart, dass eine äußere Längsfläche (7b) um die innere Längsfläche (7a) gebildet wird, wobei die innere (7a) und äußere (7b) Längsfläche eine Seitenwand (19) begrenzen, die eine seitliche Dicke hat, die überall größer als mindestens einmal die Breite (σ) des Strahls ist.

9. Strahlungseinheit nach Anspruch 8, **dadurch gekennzeichnet, dass** der Schritt des Beschneidens die Breite (σ) des Hadronenstrahls (2) derart berücksichtigt, dass die Wand (19) an mindestens einer Stelle (8) eine seitliche Dicke hat, die fünfmal kleiner als die Breite (σ) des Strahls (2) ist.

10. Strahlungseinheit nach einem der Ansprüche 8 oder 9, **dadurch gekennzeichnet, dass** die äußere Längsfläche (7b) der inneren Längsfläche (7a) entspricht.

11. Strahlungseinheit nach einem der Ansprüche 8 bis 10, **dadurch gekennzeichnet, dass** die Methode einen Schritt der Realisierung eines Stützmittels (16) umfasst, um das Abschirmungselement (1) in der Strahlungseinheit (14) zu befestigen.

12. Strahlungseinheit nach Anspruch 11, **dadurch gekennzeichnet, dass** das Stützmittel (16) ein während des Schneidschritts gebildeter Rand ist, der sich auf der äußeren Längsfläche (7b) befindet.

13. Strahlungseinheit nach Anspruch 11, **dadurch gekennzeichnet, dass** das Stützmittel (16) ein Sockel ist, der eine Öffnung aufweist, die größer oder gleich der Öffnung (11) ist mit einer Form, die der Kontur (5, 5a) entspricht und die Längsdicke (6) des Blocks (13) durchquert, um den Strahl (2) passieren zu lassen.

14. Strahlungseinheit nach Anspruch 13, **dadurch gekennzeichnet, dass** der Sockel aus Plexiglas hergestellt ist.

## Claims

1. A method for obtaining a shielding element (1) to minimize the penumbra of a hadron beam (2) outside a target zone (4), said hadron beam (2) being guided in a longitudinal direction to perform sweeping over a target zone via an irradiation unit (14), said beam (2) having a width (σ), the method comprising the following steps:
(i) defining a closed (5) or open (5a) contour of said target zone (4);
(ii) providing a block (13) having a longitudinal thickness (6) able to block the passage of said beam (2) and having a side surface (7c) perpendicular to said longitudinal thickness (6);
(iii) forming an opening (11, 17) with a shape corresponding to said contour and crossing through said longitudinal thickness (6) of said block (13) to cause said beam (2) to pass, said opening (11, 17) forming an inner longitudinal surface (7a);
(iv)trimming said block (13) so as to form a longitudinal outer surface (7b) around said inner longitudinal surface (7a), said inner longitudinal (7a) and outer longitudinal (7b) surfaces defining a side wall (19) that has a lateral thickness at least one times the width (σ) of the beam throughout;
(v) fastening said shielding element (1) in said irradiation unit (14) configured to perform sweeping of said hadron beam (2) over the target zone.

2. The method for obtaining a shielding element (1) according to claim 1, **characterized in that** the trimming step takes into account the width (σ) of the hadron beam (2) such that said wall (19) has a lateral thickness in at least one location (8) smaller than five times the width (σ) of the beam (2).

3. The method for obtaining a shielding element according to any one of the preceding claims, **characterized in that** said longitudinal outer surface (7b) corresponds to said inner longitudinal surface (7a).

4. The method for obtaining a shielding element (1) according to any one of the preceding claims, **characterized in that** it comprises a step for producing a support means (16) to fasten said shielding element (1) in said irradiation unit (14).

5. The method for obtaining a shielding element (1) according to claim 4, **characterized in that** said support means (16) is a rim situated on said outer longitudinal surface (7b) formed during said trimming step.

6. The method for obtaining a shielding element (1) according to claim 4, **characterized in that** said support means (16) is a base having an opening wider than or equal to said opening (11) with a shape corresponding to said contour (5, 5a) and crossing through said longitudinal thickness (6) of said block (13), so as to allow said beam (2) to pass.

7. The method for obtaining a shielding element (1) according to claim 6, **characterized in that** the base is made from Plexiglas.

8. An irradiation unit (14) able to sweep a hadron beam (2) from a source (3) over a target zone (4), said irradiation unit (14) comprising a shielding element (1) positioned between said source (3) and said target zone (4), so as to minimize the penumbra of the beam (2) outside said target zone (4), such shielding element being obtained using a method comprising the following steps:
(i) defining a closed (5) or open (5a) contour of said target zone (4);
(ii) providing a block (13) having a longitudinal thickness (6) able to block the passage of said beam (2) and having a side surface (7c) perpendicular to said longitudinal thickness (6);
(iii) forming an opening (11, 17) with a shape corresponding to said contour and crossing through said longitudinal thickness (6) of said block (13) to cause said beam (2) to pass, said opening (11, 17) forming an inner longitudinal surface (7a);
(iv)trimming said block (13) so as to form a longitudinal outer surface (7b) around said inner longitudinal surface (7a), said inner (7a) and outer (7b) longitudinal surfaces defining a side wall (19) that has a side thickness at least one times the width (σ) of the beam throughout.

9. The irradiation unit according to claim 8, **characterized in that** said trimming step takes into account the width (σ) of the hadron beam (2) such that said wall (19) has a lateral thickness in at least one location (8) smaller than five times the width (σ) of the beam (2).

10. The irradiation unit according to one of claims 8 or 9, **characterized in that** said outer longitudinal surface (7b) corresponds to said inner longitudinal surface (7a).

11. The irradiation unit according to any one of claims 8 to 10, **characterized in that** said method comprises a step for producing a support means (16) to fasten said shielding element (1) in said irradiation unit (14).

12. The irradiation unit according to claim 11, **characterized in that** said support means (16) is a rim situated on said outer longitudinal surface (7b) formed during said trimming step.

13. The irradiation unit according to claim 11, **characterized in that** said support means (16) is a base having an opening larger than or equal to said opening (11) with a shape corresponding to said contour (5, 5a) and crossing through said longitudinal thickness (6) of said block (13), so as to allow said beam (2) to pass.

14. The irradiation unit according to claim 13, **characterized in that** the base is made from Plexiglas.
